# EUROPÄISCHE PATENTANMELDUNG

(11) **EP 4 480 944 A1**
(43) Veröffentlichungstag der Anmeldung: **25.12.2024**
(21) Anmeldenummer: 23180222.4
(22) Anmeldetag: 20.06.2023
(51) Int. Cl.: C07C 263/10, C07C 265/14

(54) **VERFAHREN ZUR HERSTELLUNG VON ALIPHATISCHEN DIISOCYANATEN IN DER GASPHASE**

(71) Anmelder: Covestro Deutschland AG, 51373 Leverkusen (DE)
(72) Erfinder: Merkel, Michael, 40223 Düsseldorf (DE); Fischer, Christian, 42653 Solingen (DE); Beggel, Franz, 50935 Köln (DE); Krieger, Alexandra, 51065 Köln (DE); Kobylka, Manfred, 51399 Burscheid (DE)
(74) Vertreter: Levpat

(57) **Zusammenfassung**

Die Erfindung betrifft ein Verfahren zur Herstellung von aliphatischen Diisocyanaten durch Umsetzung der entsprechenden aliphatischen Diamine mit Phosgen in der Gasphase.

## Beschreibung

Die Erfindung betrifft ein Verfahren zur Herstellung von aliphatischen Diisocyanaten durch Umsetzung der entsprechenden aliphatischen Diamine mit Phosgen in der Gasphase.

Isocyanate werden in großen Mengen hergestellt und dienen hauptsächlich als Ausgangsstoffe zur Herstellung von Polyurethanen. Da die üblichen monomeren Diisocyanate in der Regel einen hohen Dampfdruck aufweisen, werden insbesondere in der Lackanwendung aus Gründen der Arbeitshygiene daraus hergestellte Polyisocyanate verwendet. Hierbei handelt es sich z.B. um Uretdione, Isocyanurate, Iminooxadiazindione, Biurete, Urethane, Allophanate oder Harnstoffe, die aus den monomeren Diisocyanaten häufig in Gegenwart von Katalysatoren hergestellt werden. Hierzu werden allerdings besonders hohe Anforderungen an die Reinheit der Monomere gestellt, da darin üblicherweise enthaltene Nebenkomponenten die Aktivität der Katalysatoren teilweise stark vermindern. Es müssen dann höhere Katalysator-Konzentrationen oder längere Reaktionszeiten angewendet werden, was die Qualität der resultierenden Polyisocyanate z.B. im Hinblick auf Farbe und Lagerstabilität deutlich verschlechtert.

Es ist daher wünschenswert, dass bereits bei der Herstellung der monomeren Diisocyanate möglichst wenig Nebenkomponenten, insbesondere chlorhaltige Nebenkomponenten, gebildet werden, um anschließend den Aufwand zu ihrer Entfernung bzw. Minimierung, z. B. durch fraktionierte Destillation, in Grenzen zu halten.

Beispiele solcher Nebenkomponenten, die einen erhöhten Aufwand in der Reinigung der monomeren Diisocyanate verursachen, stellen im Fall aliphatischer Diisocyanate die entsprechenden Chloralkylisocyanate dar, bei denen eine NCO-Gruppe durch ein CI-Atom ersetzt ist. Im Fall von 1,5-Pentandiisocyanat (im Folgenden als 1,5-PDI oder PDI abgekürzt) also beispielsweise das 5-Chlorpentylisocyanat (im Folgenden als CPI abgekürzt) und im Fall von 1,6-Hexandiisocyanat (im Folgenden als 1,6-HDI oder HDI abgekürzt) das 6-Chlorhexylisocyanat (im Folgenden als CHI abgekürzt). Einerseits senkt die Bildung dieser Chloralkylisocyanate die Ausbeute an Diisocyanat bei der Phosgenierung, andererseits wirken sie in späteren Oligo- oder Polymerisationen wegen ihrer Monofunktionalität als Kettenabbrecher. Auch ein hoher Gehalt an sogenanntem hydrolysierbarem Chlor (HC-Wert) stört bei der Weiterverarbeitung der monomeren aliphatischen Diisocyanate zu Polyisocyanaten, da es wie zuvor ausgeführt, zur Desaktivierung der üblichen Katalysatoren führt, die bei der Weiterverarbeitung eingesetzt werden, so dass allgemein monomere aliphatische Diisocyanate mit HC-Werten von < 100 ppm, bevorzugt < 50 ppm eingesetzt werden.

Die Abtrennung von Chloralkylisocyanaten wie beispielsweise CPI oder CHI vom jeweils entsprechenden Diisocyanat durch Destillation gestaltet sich schwierig, da sich deren Dampfdrücke sich meist nur wenig von denen des jeweiligen Diisocyanats unterscheiden. Es ist deshalb erstrebenswert, schon in der Rohware möglichst niedrige Gehalte an Chloralkylisocyanaten zu erzielen, um den späteren Reinigungsaufwand und damit verbundene thermische Belastungen des Produkts gering zu halten.

Die Herstellung von aliphatischen Diisocyanaten aus den entsprechenden Aminen ist an sich bekannt und kann phosgenfrei (T. Lesiak, K. Seyda, Journal für Praktische Chemie (Leipzig), 1979, 321 (1), 161 - 163) oder durch Umsetzung mit Phosgen (z.B. W. Siefken, Justus Liebigs Ann. Chem. 562, 1949, S. 25 ff., (S. 122) oder DE 2 625 075 A1) erfolgen.

Bei der oben zitierten phosgenfreien Herstellung wird das Amin zunächst mit Ameisensäure zum Formamid umgesetzt und dann mit Halogen in Gegenwart von tertiären Aminen zum Isocyanat oxidiert. Nachteilig bei diesem Verfahren ist, dass es sich um ein aufwändiges zweistufiges Verfahren handelt, wobei Nebenprodukte in beträchtlichem Ausmaß gebildet werden. Die hierdurch verursachten Ausbeuteverluste und der erforderliche hohe Reinigungsaufwand vermindern die Wirtschaftlichkeit dieses Verfahrens.

DE 2 625 075 A1 beansprucht ein Verfahren zur Herstellung von Carbamidsäurechloriden und Isocyanaten, dadurch gekennzeichnet, dass man Salze von primären Aminen in fester Form in Anwesenheit einer Flüssigkeit bei erhöhter Temperatur in einem Drehrohrofen, einem Schaufeltrockner oder in einem Wirbelbettreaktor mit Phosgen umsetzt. Nachteilig an diesem Verfahren ist, dass es sich auch hier um ein mehrstufiges Verfahren handelt, bei dem in der ersten Stufe zunächst ein Aminsalz in einem Lösungsmittel hergestellt wird, das dann vor der Umsetzung mit Phosgen, z.B. durch Filtration oder Zentrifugation und anschließender Trocknung, wieder entfernt werden muss. Dies ist zeit- und kostenaufwändig und vermindert die Wirtschaftlichkeit dieses Verfahrens.

EP 0 100 047 A1 beschreibt die Bildung von CHI als Problem bei Phosgenierung von HDA, und beschreibt als Lösung des Problems, das Hexamethylendiisocyanat statt durch Phosgenierung von HDA durch eine thermische Spaltung entsprechender Hexamethylendialkylurethane herzustellen. Zwar wird aufgrund der Abwesenheit von Chlor in diesem Verfahren kein CHI gebildet, doch erfordert das Verfahren zusätzliche Schritte für die Synthese dieser Dialkylurethane. Derartige Verfahren sind störanfällig und sowohl Aufwand als auch Ausbeute des Verfahrens sind letztlich nicht zufriedenstellend, so dass es sich im großtechnischen Maßstab nicht gegen die Phosgenierung von HDA durchsetzen konnte.

WO 2008/015134 A1 beansprucht ein Verfahren zur Herstellung von PDI, in dem biobasiertes Lysin in PDA überführt wird, welches nachfolgend in PDI überführt wird. Dabei kann die Überführung von PDA in PDI phosgenfrei oder in Gegenwart von Phosgen erfolgen, wobei die letztere Variante in der Flüssigphase oder in der Gasphase erfolgen kann. Eventuell im PDI vorhandene störende Verunreinigungen und Maßnahmen zu ihrer Vermeidung bzw. Minimierung werden nicht erwähnt.

WO 2016/042125 A1 beschreibt ein Verfahren zur Herstellung von 1,5-PDI in der Gasphase, wobei die Gas-Temperaturen der Edukte vor Eintritt in den Reaktor im Bereich von 230-320 °C liegen und die beiden Eduktströme dem Reaktor mittels einer Ringspaltdüse zugeführt werden. In den Ringspalt dieser Düse und damit zwischen die beiden Eduktströme wird dabei ein Inertgasstrom eingebracht. Mit diesem Verfahren lassen sich Rohwaren mit einem recht niedrigen Gehalt an CPI von <0,5 Gew.-%, bevorzugt <0,3 Gew.-% unter Herausrechnung des Lösungsmittels erzielen. Beispielhaft ist als niedrigster Wert ein Gehalt von 0,286 FI.-% in einer GC-Analyse offenbart.

EP 3 533 785 A1 beschreibt eine Methode zur Herstellung von 1,5-PDI, wobei das Hauptaugenmerk nicht darauf liegt, die Bildung chlorhaltiger Nebenkomponenten schon in der Reaktion zu unterdrücken, sondern vielmehr darauf, diese später durch eine Kombination von Reinigungsschritten, insbesondere umfassend einen Wärmebehandlungsschritt, zu entfernen. Für die Rohware nach dem Entgasen und dem Entfernen des Lösungsmittels sind allgemein HO-Werte von 2000 ppm bis 20000 ppm offenbart. Über das Vorhandensein der Nebenkomponente CPI oder ihre Entfernung wird nichts mitgeteilt. Angesichts der hohen HC-Werte in der Rohware liegt jedoch die Vermutung nahe, dass auch CPI in größeren Mengen entstanden sein muss. Auch dieses Verfahren erfordert zusätzliche Schritte, mindestens in Form einer Wärmebehandlung, was sich ungünstig auf seine Wirtschaftlichkeit auswirkt.

WO 2010/115908 A1 beschreibt die Herstellung von Isocyanaten in der Gasphase, wobei das Reaktionsgas unmittelbar nach der Reaktion in einem Quench durch Zugabe eines flüssigen Quenchmediums abgekühlt wird, um die Bildung von Nebenprodukten zu reduzieren bzw. zu vermeiden. Die Quenchflüssigkeit enthält dabei einen Teil des Produktstroms und wird von gegebenenfalls enthaltenen Feststoffpartikeln befreit, um Ablagerungen in Rohrleitungen und insbesondere in Zerstäuberdüsen zu vermeiden. Für die Zusammensetzung der Quenchflüssigkeit sind beliebige Zusammensetzungen zwischen 0 und 100% Isocyanat und Lösungsmittel offenbart. Eine Rohwarekonzentration oder Mengenströme aus denen auf diese Konzentration geschlossen werden könnte, werden nicht genannt. Über das Vorhandensein von Chloralkylisocyanat wird nichts offenbart und entsprechend finden sich auch keine Hinweise auf die Effekte unterschiedlicher Zusammensetzungen und Mengenströme der Quenchflüssigkeit auf die Bildung dieser Nebenkomponente.

WO 2018/224530 A1 beschreibt ein Verfahren zur Herstellung von Isocyanaten, wobei das Reaktionsproduktgemisch durch Inkontaktbringen mit einer Quenchflüssigkeit abgekühlt wird, wobei die Quenchflüssigkeit organische Lösungsmittel in einem Anteil von maximal 50 Gew.-% bezogen auf ihre Gesamtmasse umfasst und der Rest zu 100 Gew.-% aus dem herzustellenden Isocyanat besteht. Der so abgekühlte Rohproduktstrom wird in einen flüssigen und einen gasförmigen Strom getrennt und der flüssige Strom zusammen mit einem weiteren flüssigen Lösungsmittelstrom umfassend mehr als 50 Gew.-% an organischen Lösungsmitteln in einen Sammeltank eingeleitet wird. Durch diesen weiteren Lösungsmittelstrom wird der Gehalt an Lösungsmittel in der Isocyanat-Rohware, die als Quenchflüssigkeit eingesetzt wird, kontrolliert. Der Mengenstrom an Lösungsmittel ist dabei so bemessen, dass sich die gewünschte Konzentration von maximal 50 Gew.-% Lösungsmittel in der Rohware einstellt, die als Quench-Flüssigkeit in die Quenchzone zurückgeführt wird. Einen Zusammenhang zwischen der Bildung von Chloralkylisocyanaten oder Nebenprodukten ganz allgemein mit der Konzentration der Isocyanat-Rohware wird nicht hergestellt. Es wird lediglich ausgeführt, dass der Verzicht auf eine obere, überwiegend Lösungsmittel enthaltende Quenchstufe in Verbindung mit dem Einleiten des weiteren Lösungsmittelstroms in einen der Quenchzone nachgelagerten Sammeltank sich nicht negativ auf die Qualität des Produkts auswirkt. Konkrete Ausführungsbeispiele sind nicht offenbart.

WO 2018/224529 A1 beschreibt ein Verfahren zur Herstellung von Isocyanaten, wobei auch hier das Reaktionsproduktgemisch nach Durchlaufen der Reaktionszone in einer Quenchzone abgekühlt wird. Die verwendete Quenchflüssigkeit umfasst dabei maximal 25 Gew.-% an organischem Lösungsmittel. Beispielhaft sind Verfahren zur Herstellung von Toluylendiisocyanat (TDI) offenbart, bei denen im Reaktorsumpf, wo die Isocyanat-Rohware zunächst anfällt, ein Gehalt von 98 Gew.-% TDI in dieser Rohware vorliegt. Im Vergleichsbeispiel wurde an gleicher Stelle eine TDI Konzentration von 35 Gew.-% beschrieben, die Ausbeute des Prozesses ist in diesem Fall mit 97,6 % etwas geringer als für das erfindungsgemäße Verfahren. Da sich das Dokument vornehmlich mit dem aromatischen Diisocyanat TDI befasst, ist es nicht verwunderlich, dass auf die Problematik von Chloralkylisocyanaten nicht weiter eingegangen wird.

Es besteht weiterhin ein Bedarf an einem effizienten und kostengünstigen Verfahren zur Herstellung von aliphatischen Diisocyanaten mit noch niedrigeren Gehalten an Chloralkylisocyanat und vorzugsweise auch insgesamt geringen HC-Werten in der Rohware, das die Nachteile der Verfahren des Standes der Technik vermeidet.

Die Aufgabe wurde erfindungsgemäß gelöst durch ein Verfahren zur Herstellung von aliphatischen Diisocyanaten durch eine Gasphasenphosgenierung der korrespondierenden aliphatischen Diamine, wobei ein Mengenstrom mindestens eines aliphatischen Diamins mit Phosgen zu einem Reaktionsgemisch umgesetzt wird und das Reaktionsgemisch zumindest teilweise in einer Quenchzone mit einer Quenchflüssigkeit, enthaltend mindestens ein organisches Lösungsmittel, in Kontakt gebracht wird, wodurch eine Rohwarelösung erhalten wird, dadurch gekennzeichnet, dass die Quenchflüssigkeit mit einem Mengenstrom und einem Lösungsmittelgehalt in die Quenchzone eingebracht wird, die derart bemessen sind, dass die anfallende Rohwarelösung einen Gehalt an Lösungsmittel von 68 bis 88 Gew.-% aufweist.

Das erfindungsgemäße Verfahren ist insbesondere geeignet für die Herstellung von aliphatischen Diisocyanaten mit bis zu 17 Kohlenstoffatomen, vorzugsweise linearaliphatischen oder verzweigt linearaliphatischen Diisocyanaten mit 4 bis zu 11 Kohlenstoffatomen. Beispiele für solche linearaliphatischen oder verzweigt linearaliphatischen Diisocyanate sind 1,4-Butandiisocyanat, 1,5-Pentandiisocyanat (PDI), 1,6-Hexandiisocyanat (HDI), 2-Methylpentan-1,5-diisocyanat, 2,2,4-Trimethylhexan-1,6-diisocyanat, 2,4,4-Trimethylhexan-1,6-diisocyanat und 1,8-Octandiisocyanat. Besonders bevorzugte aliphatische Diisocyanate sind PDI und HDI, ganz besonders bevorzugt ist PDI, bei dem das Diisocyanat PDI und das entsprechende Chloralkylisocyanat, also 5-Chlorpentylisocyanat (CPI), bezüglich ihrer Dampfdrücke nur einen sehr geringen Unterschied aufweisen. Entsprechend groß ist für PDI der Vorteil während der Aufreinigung der PDI-Rohware zum reinen PDI, der sich aus einem geringen CPI Gehalt dieser Rohware ergibt.

Die Phosgenierung von Diaminen in der Gasphase an sich ist bekannt und kann beispielsweise erfolgen, wie beschrieben in EP 0 289 840 B1, EP 1 319 655 A2, EP 1 555 258 A1, EP 1 275 639 A1, EP 1 275 640 A1, EP 1 449 826 A1, EP 1 754 698 B1, DE 10 359 627 A1 oder DE 10 2005 042392 A1. Speziell die Phosgenierung von 1,5-Pentandiamin in der Gasphase wurde beispielsweise in der WO 2016/042125 A1 beschrieben und kann erfolgen, wie dort beschrieben.

Als Diamine können zur Durchführung des erfindungsgemäßen Verfahrens beispielsweise technische Diamine, jeweils mit einer Reinheit von > 99% und einem Wassergehalt von < 500 ppm zum Einsatz kommen. Das jeweilige Diamin kann durch bekannte Verfahren sowohl aus petrochemisch basierter Herstellung sowie auch aus biobasierter Herstellung und/oder Quelle stammen. Insbesondere für die Herstellung von PDI und HDI stammt das jeweilige Diamin oder eine Vorstufe des Diamins bevorzugt aus biobasierter Herstellung und/oder Quelle. Unter Diaminen aus biobasierter Herstellung und/oder Quelle sind vorliegend solche Diamine zu verstehen, bei deren Herstellung eine Fermentation, also eine Umsetzung mit Hilfe von Bakterien, Hefen oder Enzymen, stattgefunden hat, oder bei denen zumindest einer der Ausgangsstoffe für die Herstellung der Diamine ein nachwachsender Rohstoff ist. Besonders bevorzugt sind Diamine aus biobasierter Herstellung und Quelle, also solche, bei denen beispielsweise ausgehend von Zucker oder Aminosäuren das Diamin in einer Fermentation hergestellt wird.

Vor der Durchführung des erfindungsgemäßen Verfahrens wird das Diamin in der Regel verdampft und vorzugsweise auf 230°C bis 320°C, besonders bevorzugt auf 270°C bis 310°C, erhitzt und dann dem Reaktor, bevorzugt Rohreaktor, zugeführt. Dabei kann dem Diamin ein Inertgas wie N₂, He, Ar oder Dämpfe eines inerten Lösungsmittels, z. B. aromatischen Kohlenwasserstoffen mit oder ohne Halogensubstitution, beigemischt werden. Es ist jedoch zu beachten, dass Lösungsmitteldämpfe sich nicht immer vollständig inert verhalten, so dass als Inertgasstrom bevorzugt N₂, He oder Ar, besonders bevorzugt N₂ gewählt wird.

Das bei der Phosgenierung verwendete Phosgen wird vor der Einspeisung in den Reaktor, ebenfalls vorzugsweise auf 230°C bis 320°C, besonders bevorzugt auf 270°C bis 310°C, erhitzt. Auch dem Phosgen kann ein Inertgas wie N₂, He, Ar oder Dämpfe eines inerten Lösungsmittels, z. B. aromatischen Kohlenwasserstoffen mit oder ohne Halogensubstitution wie z.B. Chlorbenzol, o-Dichlorbenzol, Toluol, Chlortoluol, Xylol, Chlornaphtalin oder Decahydrodronaphtalin, beigemischt werden. Es ist jedoch zu beachten, dass Lösungsmitteldämpfe sich nicht immer vollständig inert verhalten, so dass als Inertgasstrom bevorzugt N₂, He oder Ar, besonders bevorzugt N₂ gewählt wird.

Ein gegebenenfalls separat zugeführter Inertgasstrom wird ebenfalls vorzugsweise auf 230°C bis 320°C, besonders bevorzugt auf 270°C bis 310°C, erhitzt. Es kann sich dabei um ein Inertgas wie N₂, He, Ar oder Dämpfe eines inerten Lösungsmittels, z. B. aromatischen Kohlenwasserstoffen mit oder ohne Halogensubstitution, handeln. Es ist jedoch zu beachten, dass Lösungsmitteldämpfe sich nicht immer vollständig inert verhalten, so dass als Inertgasstrom bevorzugt N₂, He oder Ar, besonders bevorzugt N₂ gewählt wird.

In einer bevorzugten Ausführungsform der Erfindung werden die beiden Eduktströme, sowie ein Inertgasstrom dem Reaktor mittels einer vorzugsweise konzentrisch angeordneten Ringspaltdüse zugeführt, wobei der Inertgasstrom über den Ringspalt und damit zwischen den beiden Eduktströmen zugeführt wird, während das Diamin vorzugsweise durch einen konzentrischen inneren Kanal der Ringspaltdüse und das Phosgen durch den verbleibenden Querschnitt des Reaktors zugeführt wird. Die mittlere Strömungsgeschwindigkeit des gegebenenfalls mit Inertgas verdünnten Diaminstroms beim Eintritt in den Reaktor liegt vorzugsweise im Bereich von 20-150 m/s, besonders bevorzugt im Bereich von 20-100 m/s, während das vorerhitzte und gegebenenfalls mit Inertgas verdünnte Phosgen vorzugsweise mit einer mittleren Strömungsgeschwindigkeit von mindestens 1 m/s, besonders bevorzugt von 5-15 m/s die verbleibende Querschnittsfläche zwischen Außenwand der Ringspaltdüse und der Innenwand des Reaktors passiert. Der Inertgasstrom, der Diamin und Phosgen beim Eintritt in den Reaktor kurz trennt, kann beispielsweise mit einer mittleren Strömungsgeschwindigkeit von 20-150 m/s, vorzugsweise 20-100 m/s, dem Reaktor zugeführt werden.

Die vorzugsweise zu verwendende Ringspaltdüse wird, da der Inertgasstrom die beiden Eduktströme trennt, im Folgenden auch als Trennspaltdüse bezeichnet.

Die Mengenströme von gasförmigem Diamin und Phosgen werden vorzugsweise so gewählt, dass der molare Phosgenüberschuss bezogen auf eine Aminogruppe 30 bis 300 %, bevorzugt 60 bis 200 % beträgt.

Bevorzugt werden im erfindungsgemäßen Verfahren Rohrreaktoren ohne Einbauten und ohne sich bewegende Teile im Innern des Reaktors verwendet. Die Rohrreaktoren bestehen im Allgemeinen aus Stahl, Glas, legiertem oder emailliertem Stahl und sind so dimensioniert, dass unter den Verfahrensbedingungen eine weitgehend vollständige Umsetzung, bevorzugt eine vollständige Umsetzung des Diamins mit dem Phosgen ermöglicht wird. Die Gasströme werden wie oben beschrieben beispielsweise über eine Trennspaltdüse an einem Ende des Rohrreaktors in diesen eingeleitet. Die Mischzone wird bevorzugt bei einer Temperatur innerhalb des Bereiches von 230°C bis 320°C, vorzugsweise 270°C bis 310°C, gehalten, wobei diese Temperatur gegebenenfalls und bei Bedarf durch Beheizung des Rohreaktors aufrechterhalten werden kann.

Die getrennt aufgeheizten Reaktionspartner Phosgen und aliphatisches Diamin werden vermischt und umgesetzt, wobei der Bereich des Reaktors, in dem die Umsetzung erfolgt, als Reaktionszone definiert ist. Die Umsetzung in der Reaktionszone erfolgt bevorzugt adiabatisch, also ohne gezielte Abfuhr der freigesetzten Reaktionswärme. Bevorzugt bleibt dabei jedoch die Temperatur in der Reaktionszone unterhalb von 450 °C, besonders bevorzugt unterhalb von 425 °C. Dies kann durch die Mengenströme an Diamin, Phosgen und gegebenenfalls Inertgas, sowie deren Eintrittstemperaturen gesteuert werden.

Mischung und Reaktion erfolgen bevorzugt in einer gemeinsamen technischen Vorrichtung zur Durchführung chemischer Reaktionen, dem Reaktor. Der Bereich, in dem die Mischung erfolgt, wird dabei Mischzone und der Bereich, in dem die Reaktion erfolgt, Reaktionszone genannt. Mischzone und Reaktionszone sind dabei nicht klar räumlich voneinander abzugrenzen, sondern gehen fließend ineinander über und überlappen sich entsprechend räumlich.

Bei der Durchführung des erfindungsgemäßen Verfahrens liegt im Allgemeinen der Druck in den Zuleitungen zum Reaktionsraum bei 200 bis 3.000 mbar abs., bevorzugt bei 800 bis 1.500 mbar abs., und am Ausgang aus dem Reaktionsraum bei 150 - 2.000 mbar abs., bevorzugt bei 750 bis 1440 mbar abs., wobei durch Aufrechterhaltung eines geeigneten Differenzdrucks eine Strömungsgeschwindigkeit innerhalb des Reaktionsraums von 3 bis 120 m/s, bevorzugt 5 bis 75 m/s eingehalten wird. Unter diesen Voraussetzungen herrschen innerhalb des Reaktionsraums im Allgemeinen turbulente Strömungsverhältnisse vor.

Die mittlere Verweilzeit des Reaktionsgemischs im Reaktor beträgt beispielsweise 0,05 bis 4 s, bevorzugt 0,1 bis 2 s, besonders bevorzugt 0,2 bis 0,5 s. Die mittlere Verweilzeit berechnet sich aus dem zeitlichen Durchsatz der Eduktströme, der Reaktordimensionierung und den Reaktionsparametern Druck und Temperatur.

Nach dem Durchlaufen der Reaktionszone wird zumindest ein Teil, bevorzugt jedoch das gesamte erhaltene Reaktionsgemisch, rasch abgekühlt und dabei unter Erhalt einer Rohwarelösung zumindest teilweise kondensiert. Dies erfolgt, indem es in eine Quenchzone eingebracht wird und dort mit einer Quenchflüssigkeit in Kontakt gebracht wird. Aufbau und Betrieb einer Quenchzone im Rahmen der Herstellung von Diisocyanaten durch Phosgenierung der entsprechenden Diamine in der Gasphase sind dem Fachmann hinlänglich bekannt. Mögliche Ausgestaltungen sind beispielsweise in EP 1 403 248 A1 und in EP 1 935 875 A1 offenbart.

In einer bevorzugten Ausführungsform sind Mischzone, Reaktionszone und Quenchzone in einer gemeinsamen technischen Vorrichtung, dem Reaktor, angeordnet.

Die eingesetzte Quenchflüssigkeit enthält mindestens ein organisches Lösungsmittel umfassend bevorzugt aliphatische Kohlenwasserstoffe, aromatische Kohlenwasserstoffe ohne Halogensubstitution, aromatische Kohlenwasserstoffe mit Halogensubstitution und Mischungen der vorgenannten Lösungsmittel. Bevorzugt ist das in der Quenchflüssigkeit enthaltene, mindestens eine organische Lösungsmittel Chlorbenzol, p-Dichlorbenzol, o-Dichlorbenzol, Chlortoluol und Chlornaphthalin oder Mischungen der vorgenannten Lösungsmittel. Besonders bevorzugt ist das in der Quenchflüssigkeit enthaltene, mindestens eine organische Lösungsmittel Chlorbenzol und o-Dichlorbenzol oder eine Mischung der vorgenannten Lösungsmittel. Ganz besonders bevorzugt ist das in der Quenchflüssigkeit enthaltene, mindestens eine organische Lösungsmittel Chlorbenzol. Bevorzugt enthält die Quenchflüssigkeit neben dem Lösungsmittel auch das bei der Reaktion gebildete Isocyanat, da bevorzugt zumindest ein Teil der Quenchflüssigkeit aus der anfallenden Isocyanat-Rohware entnommen wird. In diesem Fall ist es zur Vermeidung von Feststoffablagerungen in Rohleitungen, Ventilen, Düsen oder anderen Einrichtungen des Quenchs empfehlenswert, gegebenenfalls in der Quenchflüssigkeit enthaltene Feststoffpartikel abzutrennen. Maßnahmen, mit denen eine solche Feststoffabtrennung erfolgen kann, sind dem Fachmann bekannt, bevorzugt erfolgt eine Filtration zumindest eines Teils der Quenchflüssigkeit.

Um den erfindungsgemäßen Anteil an Lösungsmittel in der anfallenden Rohware-Lösung von 68 bis 88 Gew.-% zu erreichen, ist es zweckmäßig, dass die Quenchflüssigkeit selbst einen Lösungsmittelgehalt oberhalb des dieses Zielwerts aufweist. Der Lösungsmittelgehalt der Quenchflüssigkeit liegt vorzugsweise bei wenigstens 70 Gew.-%, bevorzugt wenigstens 72 Gew.-% und besonders bevorzugt wenigstens 75 Gew.-%. Sofern eine Quenche mit zwei oder mehr Stufen und möglicherweise unterschiedlicher Quenchflüssigkeit zum Einsatz kommt, ist hierbei der rechnerische Lösungsmittelgehalt der insgesamt eingesetzten Quenchflüssigkeit, also der Summe aller Quenchflüssigkeits-Ströme, wesentlich. Sofern mehr als ein Lösungsmittel in der Quenchflüssigkeit enthalten ist, betrifft der Lösungsmittelgehalt die Gesamtheit der Lösungsmittel.

In einer bevorzugten Ausführungsform der Erfindung werden der Mengenstrom und der Lösungsmittelgehalt der Quenchflüssgikeit derart bemessen, dass die anfallende Rohwarelösung einen Gehalt an Lösungsmittel von 70 bis 86 Gew.-%, bevorzugt von 72 bis 85 Gew.-% und besonders bevorzugt von 74 bis 84 Gew.-% aufweist. Höhere Gehalte sind dabei vorteilhaft für einen besonders niedrigen Gehalt an Chloralkylisocyanaten in der Rohware. Zu hohe Lösungsmittelgehalte verursachen andererseits einen erhöhten Aufwand für die Abtrennung und Rückführung des Lösungsmittels, so dass auf eine allzu starke Verdünnung der Rohware verzichtet werden sollte.

Die Anfallende Rohware kann in einem Sammeltank mit weiterem Lösungsmittel weiter verdünnt werden, jedoch ist das angesichts des ohnehin schon hohen Lösungsmittelgehalts der Rohware in der Regel weder erforderlich noch vorteilhaft. Es wirkt sich vielmehr negativ auf den Gesamtprozess aus, da dieses zusätzliche Lösungsmittel später wieder vom Isocyanat abgetrennt werden müsste. Eine Ausnahme hiervon bilden Verfahren, bei denen zumindest ein Teil der Rohware-Lösung aus dem Sammeltank entnommen und selbst als Quenchflüssigkeit eingesetzt wird. In diesem Fall erhöht das weitere, in den Sammeltank zugegebene Lösungsmittel den Lösungsmittelgehalt im Quench-Medium, so dass über diese Zugabe an weiterem Lösungsmittel in den Sammeltank der Lösungsmittelgehalt in der anfallenden Rohware beeinflusst und eingestellt werden kann. Sofern keine Beprobung der anfallenden Rohware erfolgen kann, bevor deren Zusammensetzung weiter verändert wird, oder einfach um den Aufwand für Prozessanalytik zu reduzieren, ist es dem Fachmann auch möglich, den Gehalt an Lösungsmittel in der anfallenden Rohware rechnerisch zu ermitteln. Eine derartige Berechnung kann auch vorteilhaft eingesetzt werden, um den erforderlichen Mengenstrom an Quenchflüssigkeit in die Quenchzone und deren Lösungsmittelgehalt zu bemessen.

In einer bevorzugten Ausführungsform des Verfahrens werden der Mengenstrom und/oder der Lösungsmittelgehalt der Quenchflüssigkeit computerimplementiert bemessen, wobei anhand von Betriebsparametern ein erwarteter Gehalt an Lösungsmittel in der Rohwarelösung errechnet und in mindestens eine Stellgröße für mindestens einen Aktor übersetzt wird, der den Mengenstrom und/oder den Lösungsmittelgehalt der Quenchflüssigkeit beeinflusst. Beispiele für solche Aktoren sind Pumpen oder Ventile, bevorzugt Regelventile, die den Mengenstrom der Quenchflüssigkeit regeln oder die den Eintrag von frischem oder aufbereitetem Lösungsmittel in die Quenchflüssigkeit regeln und somit den Lösungsmittelgehalt der Quenchflüssigkeit und in der Folge auch den Gehalt an Lösungsmittel in der Rohware beeinflussen. Ein weiterer Gegenstand der vorliegenden Erfindung ist ein computerimplementiertes Verfahren zur Bemessung des erfindungsgemäß einzubringenden Mengenstroms, dadurch gekennzeichnet, dass anhand von Daten und Parametern die Rohwarekonzentration während der Herstellung von aliphatischen Diisocyanaten durch Gasphasenphosgenierung der korrespondierenden aliphatischen Diamine rechnerisch ermittelt wird.

Die Quenchzone kann so ausgestaltet sein, dass die Zugabe von Quenchflüssigkeit an nur einer Position in Längsrichtung des Reaktors erfolgt oder aber so, dass die Zugabe von Quenchflüssigkeit an zwei oder mehr Positionen in Längsrichtung des Reaktors erfolgt. Derartige unterschiedliche Positionen in Längsrichtung des Reaktors mit Quenchflüssgikeitszugabe werden im Folgenden auch als Quenchstufen bezeichnet. Jede Quenchstufe ist vorzugsweise mit mehreren Düsen versehen, die entlang des Reaktorumfangs verteilt sind. Bevorzugt umfasst die Quenchzone zwei oder mehr, besonders bevorzugt genau zwei Quenchstufen. In dieser Ausgestaltung können die unterschiedlichen Quenchstufen mit unterschiedlichen Quenchflüssigkeiten beschickt werden, also Quenchflüssigkeiten unterschiedlicher Zusammensetzung und damit auch unterschiedlichen Lösungsmittelgehalts. Vorzugsweise wird in am weitesten stromabwärts gelegener Position, bei vertikal angeordneten Rohrreaktoren also der untersten Position, eine Quenchflüssigkeit mit dem niedrigsten Lösungsmittelgehalt zugegeben, während an weiter stromaufwärts gelegenen Positionen Quenchflüssigkeiten mit einem höheren Lösungsmittelgehalt eingesetzt werden.

Die Temperatur der Quenchflüssigkeit wird vorzugsweise so gewählt, dass sie einerseits oberhalb der Zersetzungstemperatur des dem Diisocyanat entsprechenden Carbamidsäurechlorids und andererseits unterhalb der Kondensationstemperatur des Diisocyanats unter den Prozessbedingungen in der Quenchzone liegt. Sofern Lösungsmitteldämpfe als verdünnendes Inertgas zugesetzt wurden, liegt die Temperatur der Quenchflüssigkeit vorzugsweise auch unterhalb der Kondensationstemperatur dieses Lösungsmittels, so dass Diisocyanat und Hilfslösungsmittel kondensieren bzw. sich in der Quenchflüssigkeit lösen, während überschüssiges Phosgen, Chlorwasserstoff und gegebenenfalls Teile von Lösungsmitteln und verdampfter Quenchflüssigkeit die Quenchzone gasförmig verlassen. Zur selektiven Gewinnung des Diisocyanats aus dem die Reaktionszone gasförmig verlassenden Gemisch besonders gut geeignet sind insbesondere bei einer Temperatur von 60 bis 200°C, vorzugsweise 90 bis 170°C, gehaltene Quenchflüssigkeiten.

Die die Quenchzone gasförmig verlassenden Anteile des Reaktiongemisches werden anschließend in an sich bekannter Weise von überschüssigem Phosgen befreit. Dies kann mittels einer Kühlfalle, Absorption in einem bei einer Temperatur von -10°C bis 8°C gehaltenen inerten Lösungsmittel (z.B. Chlorbenzol, MCB, oder Dichlorbenzol, ODB) oder durch Adsorption und Hydrolyse an Aktivkohle erfolgen. Das die Phosgen-Rückgewinnungsstufe durchlaufende Chlorwasserstoffgas kann in an sich bekannter Weise zur Rückgewinnung des zur Phosgensynthese erforderlichen Chlors rezykliert werden.

Die Reindarstellung des Diisocyanats erfolgt vorzugsweise durch destillative Aufarbeitung der Isocyanat-Rohwarelösung.

Die Vorteile des erfindungsgemäßen Verfahrens sind:
a) Geringe Nebenproduktbildung und dadurch niedrige Gehalte an chlorhaltigen Nebenprodukten, insbesondere Chloralkylisocyanaten, bereits in den Rohwaren. Unter Herausrechnung des Lösungsmittels liegen die Konzentrationen von Chloralkylisocyanat in der Rohwarelösung bei weniger als 0,25 Gew.-%. Dadurch kann der Aufwand bei der nachfolgenden Destillation gering gehalten werden.
b) Vermeidung von festen Ablagerungen an der Reaktorwand und in der Quenche.

Ein weiterer Gegenstand der vorliegenden Erfindung ist eine Vorrichtung zur Herstellung von aliphatischen Diisocyanaten mittels Gasphasenphosgenierung nach dem erfindungsgemäßen Verfahren, umfassend
- einen Reaktor mit wenigstens einer Reaktionszone, die zur Umsetzung mindestens eines aliphatischen Diamins mit Phosgen eingerichtet ist, sowie wenigstens einer Quenchzone, die zur Kontaktierung des Reaktionsgemisches mit einer Quenchflüssigkeit enthaltend mindestens ein organisches Lösungsmittel eingerichtet ist,
- wenigstens einen Aktor, der dazu eingerichtet ist, den Mengenstrom und/oder den Lösungsmittelgehalt der Quenchflüssigkeit zu beeinflussen,
- eine Schnittstelleneinheit, die dazu eingerichtet ist, wenigstens einen Betriebsparameter des Reaktors kontinuierlich oder periodisch einzulesen
- einen Prozessor, der dazu eingerichtet ist, den wenigstens einen kontinuierlich oder periodisch eingelesenen Betriebsparameter zu verarbeiten, daraus einen theoretischen Lösungsmittelgehalt für die anfallende Rohware zu errechnen und bei einer Abweichung dieses Lösungsmittelgehalts von einem vorgegebenen Zielwert für diesen Lösungsmittelgehalt entweder einen Hinweis auf einer Anzeige auszugeben oder eine neue Stellgröße an den wenigstens einen Aktor zu übermitteln und so den Lösungsmittelgehalt in der anfallenden Rohware anzupassen.

Ebenfalls Gegenstand der vorliegenden Erfindung ist eine Verwendung einer Quenchflüssigkeit mit derart bemessenem Mengenstrom und Lösungsmittelgehalt, dass der Chloralkylisocyanat-Gehalt aliphatischer Diisocyanate reduziert wird.

Im Folgenden wird die Erfindung an Beispielen näher beschrieben, ohne jedoch auf diese beschränkt zu sein.

### Beispiele:

### GC-Methode für die Rohware-Analytik:

| | | |
|---|---|---|
| Gas-Chromatograph: | Agilent ( ehemals Hewlett PACKARD ), 7890, Serie A oder B ( 6890 Serie A oder B sind auch möglich ), | |
| Trennsäule: | RXI 17 (Restek), fused Silica, Länge 30 m, innerer Durchmesser 0,32 mm, Filmdicke 1,0 µm | |
| Temperaturen: | Injektor 250°C, Detektor (FID) 350°C, | |
| | Ofen: | Start 80°C, Haltezeit 0 min , |
| | | Heizrate 10°K/min auf 140°C, Haltezeit 7,5 min |
| | | Heizrate 20°K/min auf 250°C, Haltezeit 5,0 min |
| | | Laufzeit 24 min. |
| Trägergas: | Wasserstoff | |
| | Gaseinstellung | konstanter Fluss anstatt konstanter Druck |
| | Säulendruck | ca. 0,4 bar abs., bei Start der Analyse |
| | Säulenströmung | ca. 100mL/min bei konstantem Fluss |
| | Splitausgang | Fluss 100mL/ min |
| | | Verhältnis 50:1 |
| | Septumspülung | ca. 3 ml/min |

Die quantitative Auswertung erfolgt über die normierte Flächenprozent-Methode. Für das jeweilige Lösungsmittel wird über eine Kalibrierung mit externem Standard ein Response Faktor im erwarteten Konzentrationsbereich bestimmt, der bei der Ermittlung des Lösungsmittelgehalts in der Rohwarelösung berücksichtigt wird. Für Chloralkylisocyanate und Diisocyanate wird ein Response-Faktor von 1 zur Ermittlung der normierten Flächenprozente angenommen.

### Vergleichsbeispiel 1:

PDA wurde verdampft und auf 310 °C überhitzt und so ein Strom von 8,46 kg/h an gasförmigem PDA bereitgestellt. Gleichzeitig wurden ein Strom von 45 kg/h Phosgen bei 310 °C bereitgestellt und ein Strom von 1,48 kg/h Stickstoff bei 310 °C bereitgestellt. Diese Ströme wurden einem vertikal angeordneten, konisch-zylindrischen Reaktor zugeführt, welcher Mischzone, Reaktionszone, Quenchzone und einen Sammeltank umfasste. Die Zugabe erfolgte am oberen Ende des Reaktors, wobei das PDA durch ein koaxial verlaufendes Zentralrohr einer Trennspaltdüse zugegeben wurde, und der Stickstoff durch den Trennspalt dieser Düse. Phosgen strömte durch den verbleibenden Ringraum zwischen Düse und innerer Reaktorwand an der Düse vorbei. Am Austritt der Düse war somit der Stickstoffstrom zwischen dem Amin- und dem Phosgenstrom angeordnet. Stromabwärts erfolgte spontan die Mischung der Stoffströme und das Reaktionsgemsich durchlief unter Bildung des Isocyanats die Reaktionszone. Das so erhaltene Reaktionsgemisch wurde weiter stromabwärts in einem zweistufig ausgeführten Quench durch Eindüsen von Quenchflüssigkeiten abgekühlt, woraufhin ein Teil des Reaktionsgemisches kondensierte und frei in einen unterhalb des Quenchs befindlichen Sammeltank ablief. Der hierbei nicht niedergeschlagene Teil des Reaktionsgemisches verließ den Reaktor gasförmig und wurde in einer mit Chlorbenzol betriebenen Auswaschkolonne von Isocyanat-Resten befreit. Die anfallende Waschlösung enthielt ca. 98 Gew.-% Chlorbenzol sowie geringe Mengen an Phosgen und PDI.

Die Quenchflüssigkeit für die erste, obere Quenchstufe wurde aus dieser Waschlösung entnommen, während in der zweiten, unteren Quenchstufe als Quenchflüssigkeit ein dem Sammeltank entnommener Rohwarestrom in die Quenchzone eingedüst wurde, so dass letztlich die Isocyanat-Rohware über den Sammeltank und die zweite untere Quenchstufe zirkuliert wurde.

Der Mengenstrom an Quenchflüssigkeit zur ersten Quenchstufe wurde so eingestellt, dass in der im Sammeltank anfallenden Rohware ein Chlorbenzolgehalt von 65 Gew.-% vorlag.

Die GC-Analytik der erhaltenen Rohlösung zeigte einen CPI-Gehalt von 0,48 Gew.-% bezogen auf die Rohware ohne Lösungsmittel.

### Beispiel 1:

Die Phosgenierung wird wie in Vergleichsbeispiel 1 durchgeführt, jedoch wurde der Mengenstrom zur ersten Quenchstufe erhöht, so dass der Gehalt an Chlorbenzol in der Rohware bei 68 Gew.-% lag.

Die GC-Analytik der erhaltenen Rohlösung zeigte einen CPI-Gehalt von 0,22 Gew.-% bezogen auf die Rohware ohne Lösungsmittel.

### Beispiel 2:

Die Phosgenierung wird wie in Vergleichsbeispiel 1 durchgeführt, jedoch wurde über eine zusätzliche Einspeisung von Chlorbenzol in den Sammeltank die der Gehalt an Chlorbenzol in der Rohware und (und somit auch in der Quenchflüssigkeit zur zweiten, unteren QuenchStufe) auf 70 Gew.-% eingestellt.

Die GC-Analytik der erhaltenen Rohlösung zeigte einen CPI-Gehalt von 0,16 Gew.-% bezogen auf die Rohware ohne Lösungsmittel.

### Beispiel 3:

Die Phosgenierung wird wie in Vergleichsbeispiel 1 durchgeführt, jedoch wurde der Mengenstrom zur ersten Quenchstufe erhöht, so dass der Gehalt an Chlorbenzol in der Rohware bei 74,5 Gew.-% lag.

Die GC-Analytik der erhaltenen Rohlösung zeigte einen CPI-Gehalt von 0,09 Gew.-% bezogen auf die Rohware ohne Lösungsmittel.

### Beispiel 4:

Die Phosgenierung aus Beispiel 2 wurde wiederholt, wobei nun unter sonst gleichen Bedingungen nicht PDA, sondern ein Strom von 9,62 kg/h an HDA phosgeniert wurde. Der Lösungsmittelgehalt der Rohwarelösung wurde wie in Beispiel 2 auf 70 Gew.-% eingestellt.

Die GC-Analytik der erhaltenen Rohlösung zeigte einen CHI-Gehalt von 0,17 Gew.-% bezogen auf die Rohware ohne Lösungsmittel.

## Patentansprüche

1. Verfahren zur Herstellung von aliphatischen Diisocyanaten durch eine Gasphasenphosgenierung der korrespondierenden aliphatischen Diamine,
• wobei ein Mengenstrom mindestens eines aliphatischen Diamins mit Phosgen zu einem Reaktionsgemisch umgesetzt wird und
• das Reaktionsgemisch zumindest teilweise in einer Quenchzone mit einer Quenchflüssigkeit, enthaltend mindestens ein organisches Lösungsmittel, in Kontakt gebracht wird, wodurch eine Rohwarelösung erhalten wird,
**dadurch gekennzeichnet, dass** die Quenchflüssigkeit mit einem Mengenstrom und einem Lösungsmittelgehalt in die Quenchzone eingebracht wird, die derart bemessen sind, dass die Rohwarelösung einen Gehalt an Lösungsmittel von 68 bis 88 Gew.-% aufweist.

2. Verfahren gemäß Anspruch 1, **dadurch gekennzeichnet, dass** das aliphatische Diisocyanat 1,5-Pentandiisocyanat ist.

3. Verfahren gemäß Anspruch 1 oder 2, **dadurch gekennzeichnet, dass** das mindestens eine aliphatische Diamin 1,5-Pentandiamin ist, bevorzugt 1,5-Pentandiamin ist und aus biobasierter Herstellung und/oder Quelle stammt.

4. Verfahren gemäß einem der Ansprüche 1 bis 3, **dadurch gekennzeichnet, dass** das mindestens eine Lösungsmittel Chlorbenzol, p-Dichlorbenzol, o-Dichlorbenzol, Chlortoluol Chlornaphthalin oder eine Mischung der vorgenannten Lösungsmittel ist.

5. Verfahren gemäß einem der Ansprüche 1 bis 4, **dadurch gekennzeichnet, dass** der Lösungsmittelgehalt der Quenchflüssigkeit bei wenigstens 70 Gew.-%, bevorzugt wenigstens 72 Gew.-% und besonders bevorzugt bei wenigstens 75 Gew.-% liegt.

6. Verfahren gemäß einem der Ansprüche 1 bis 5, **dadurch gekennzeichnet, dass** der eingebrachte Mengenstrom und der Lösungsmittelgehalt der Quenchflüssigkeit derart bemessen sind, dass die anfallende Rohwarelösung einen Gehalt an Lösungsmittel von 70 bis 86 Gew.-%, bevorzugt von 72 bis 85 Gew.-% und besonders bevorzugt von 74 bis 84 Gew.-% aufweist.

7. Verfahren gemäß einem der Ansprüche 1 bis 6, **dadurch gekennzeichnet, dass** die Quenchzone zwei oder mehr, bevorzugt zwei Quenchstufen umfasst.

8. Verfahren gemäß einem der Ansprüche 1 bis 7, **dadurch gekennzeichnet, dass** der Mengenstrom und/oder der Lösungsmittelgehalt der Quenchflüssigkeit computerimplementiert bemessen werden, wobei anhand von Betriebsparametern ein erwarteter Gehalt an Lösungsmittel in der Rohwarelösung errechnet und in mindestens eine Stellgröße für mindestens einen Aktor übersetzt wird, der den Mengenstrom und/oder den Lösungsmittelgehalt der Quenchflüssigkeit beeinflusst.

9. Vorrichtung zur Herstellung von aliphatischen Diisocyanaten mittels Gasphasenphosgenierung gemäß einem der Ansprüche 1 bis 7, umfassend
• einen Reaktor mit wenigstens einer Reaktionszone, die zur Umsetzung mindestens eines aliphatischen Diamins mit Phosgen eingerichtet ist, sowie wenigstens einer Quenchzone, die zur Kontaktierung des Reaktionsgemisches mit einer Quenchflüssigkeit enthaltend mindestens ein organisches Lösungsmittel eingerichtet ist,
• wenigstens einen Aktor, der dazu eingerichtet ist, den Mengenstrom und/oder den Lösungsmittelgehalt der Quenchflüssigkeit zu beeinflussen,
• eine Schnittstelleneinheit, die dazu eingerichtet ist, wenigstens einen Betriebsparameter des Reaktors kontinuierlich oder periodisch einzulesen
• einen Prozessor, der dazu eingerichtet ist, den wenigstens einen kontinuierlich oder periodisch eingelesenen Betriebsparameter zu verarbeiten, daraus einen theoretischen Lösungsmittelgehalt für die anfallende Rohware zu errechnen und bei einer Abweichung dieses Lösungsmittelgehalts von einem vorgegebenen Zielwert für diesen Lösungsmittelgehalt entweder einen Hinweis auf einer Anzeige auszugeben oder eine neue Stellgröße an den wenigstens einen Aktor zu übermitteln und so den Lösungsmittelgehalt in der anfallenden Rohware anzupassen.

10. Verwendung einer Quenchflüssigkeit mit derart bemessenem Mengenstrom und Lösungsmittelgehalt, dass der Chloralkylisocyanat-Gehalt aliphatischer Diisocyanate reduziert wird.

11. Computerimplementiertes Verfahren zur Bemessung des gemäß einem der Ansprüche 1 bis 8 eingebrachten Mengenstroms, **dadurch gekennzeichnet, dass** anhand von Daten und Parametern die Rohwarekonzentration während der Herstellung von aliphatischen Diisocyanaten durch Gasphasenphosgenierung der korrespondierenden aliphatischen Diamine rechnerisch ermittelt wird.
